# EUROPÄISCHE PATENTANMELDUNG

(11) **EP 4 838 216 A1**
(43) Veröffentlichungstag der Anmeldung: **11.11.2026**
(21) Anmeldenummer: 25174194.8
(22) Anmeldetag: 05.05.2025
(51) Int. Cl.: G01N 33/202, B01D 53/46, C22B 7/02, G01N 15/06, G01N 21/59, G01N 21/71

(54) **ANALYSESYSTEM UND VERFAHREN ZUR ERMITTLUNG VON AUSGETRAGENEN ZUSCHLAGSTOFFEN EINES METALLURGISCHEN PROZESSES**

(71) Anmelder: Primetals Technologies Austria GmbH, 4031 Linz (AT)
(72) Erfinder: Fischer, Paul, 4048 Puchenau (AT); Mayrhofer, Anna, 4722 Peuerbach (AT); Reiter, Alexander, 4040 Lichtenberg (AT)
(74) Vertreter: Metals@Linz

(57) **Zusammenfassung**

Die vorliegende Erfindung betrifft das Gebiet der metallurgischen Anlagen, konkret metallurgische Anlagen in welchen Zuschlagstoffe (5) in einen metallurgischen Prozess eingebracht werden und über eine Entstaubungsanlage austretende Abgas mit Staub (6a) des metallurgischen Prozesses abgesaugt werden und der Staub (6a) vom Abgas abgetrennt wird.

Die Aufgabe der Erfindung ist es den - über die Prozessabsaugung - mitgerissenen variablen Feinanteil messbar zu machen und dadurch den Prozess effizienter zu gestalten. Die Aufgabe wird gelöst durch ein Analysesystem (12) welches eine Zuführeinheit (11), zum Zuführen vom abgetrennten Staub (6a) zu einem Materialanalysegerät (13) aufweist. Das Materialanalysegerät (13) ist zum Bestimmen der Anteile der im abgetrennten Staub (6a) enthaltenen Stoffe, insbesondere der Zuschlagsstoffe (5), ausgelegt. Das Analysesystem (12) umfasst weiters eine Masseermittlungsvorrichtung (14), welche zur Bestimmung einer Masse des abgetrennten Staubes (6a) ausgestaltet ist. Eine Auswerteeinrichtung (15), welche mit der Materialanalysegerät (13), der Masseermittlungsvorrichtung (14) und der Zuführeinheit (11) verbunden ist, ermittelt die Zuschlagstoffmasse, die im abgetrennten Staub (6a) enthalten ist.

## Beschreibung

### Gebiet der Technik

Die vorliegende Erfindung betrifft das Gebiet der metallurgischen Anlagen, konkret metallurgische Anlagen in welchen Zuschlagstoffe in einen metallurgischen Prozess eingebracht werden und über ein Entstaubungsanlage austretende Abgase und Staub des metallurgischen Prozesses abgesaugt werden und der Staub vom Abgas abgetrennt wird. Einerseits betrifft die Erfindung ein Analysesystem zur Ermittlung zumindest einer Zuschlagstoffmasse von einem ausgetragenen Zuschlagsstoff eines metallurgischen Prozesses, wobei dem Analysesystem ein durch ein Abscheidungssystem von einem Abgas abgetrennter Staub zugeführt werden kann.

Andererseits betrifft die Erfindung ein Verfahren zur Ermittlung zumindest einer Zuschlagstoffmasse von zumindest einem ausgetragenen Zuschlagsstoff eines metallurgischen Prozesses. Dem metallurgischen Prozess wird mittels eines Absaugsystem ein mit Staub beladener Abgasstrom abgezogen und an einem Abscheidungssystem wird der Staub vom Abgasstrom abgetrennt.

Des Weiteren betrifft die Erfindung eine Metallurgische Anlage zum Herstellen eines flüssigen Metalls, umfassend ein Beschickungssystem für zumindest einen Zuschlagstoff, eine Entstaubungsanlage mit einem Abscheidungssystem für ausgetragenem Staub und einer Prozesssteuerung und/oder Prozessregelung

### Stand der Technik

Bei metallurgischen Prozessen, vor allem bei einem Sauerstoffaufblas-Konverter-Prozess - LD (Linz Donawitz) -Verfahren - werden aus metallurgischen und prozesstechnischen Gründen Zuschlagsstoffe verwendet. Diese Zuschlagsstoffe werden meist in Form von Feststoffen - über Förderbänder, Bunker, Schurren - dem Prozess zugeführt. Diese Feststoffe verfügen über eine gewisse Korngrößenverteilung. Durch hohe Gasgeschwindigkeiten eines Gasstromes des metallurgischen Prozesses selbst werden Teile der Zuschlagsstoffe während des Zuführens als Staub direkt abgesaugt, bevor sie den Prozess selbst erreichen können und ihre eigentlich bestimmte chemische/metallurgische Reaktion erfüllen können. Dieser Anteil der Zuschlagsstoffe, der von dem Gasstrom mitgerissen wird, ist als Menge unbekannt. Je nach Material werden typische Werte als konstant betrachtet und so für Optimierungen verwendet. Durch Variationen von angelieferten Materialien - bspw. durch verschiedene Lieferanten -, Segregation im Materialtransport und in Materialbunkern kommt es aber zu einer zeitlichen Variation in den jeweiligen Feinstoffanteilen und dementsprechend ist der Feinanteil, der über eine Entstaubungsanalage mitgerissen wird, variabel.

Die Zugabe von Materialien findet nicht nur beim LD-Verfahren statt, sondern auch bei anderen metallurgischen Prozessen wie beispielsweise einem Hochofen, ElektroLichtbogenofen oder einem Smelter.

Diese metallurgischen Prozesse werden durch eine Bedienperson oder ein Automationssystem überwacht und gesteuert. Der Einsatz eines Level-2-Prozeßrechners ermöglicht es, flexibel und schnell auf sich ändernde Einsatzstoffe und Ziele zu Blasende - vor einem Abstich bzw. während dem Abstich - zu reagieren und den Produktionsprozess zu stabilisieren. Ziele sind beispielsweise Menge, Temperatur und chemische Analyse des füßigen Metalls. Der Bediener wird durch die verschiedenen Prozess-Schritte während der Schmelze geführt und angeleitet, um eine konsistente und reproduzierbare Produktion mit Erreichung der Produktionsziele zu gewährleisten. Er wird von einer Benutzeroberflache unterstützt, um die Produktion einer Schmelze zu starten und zu überwachen bzw. zu steuern. Grundlage aller Berechnungen sind metallurgische Modelle, die die Eigenschaften der Schmelze während der gesamten Behandlung modellieren. Analysemodell und Thermisches Modell sind die wichtigsten Modelle, in welchen Regeln von Massenbilanz, Thermodynamik und Reaktionskinetik angewendet werden. Zyklisch werden aktuelle Reaktionen berechnet. Das beinhaltet ein Schmelzen bzw. Auflösen von Schrott und Materialzugaben, sowie Oxidations- und Reduktionsreaktionen. Eine aktuelle Temperatur wird zyklisch über die thermische Bilanz berechnet, wobei eine Reaktionsenthalpie und thermische Verluste berücksichtigt werden. Der Level 2 Prozessrechner schlägt die erforderlichen Aktionen des Bedieners sowie des Prozessautomationssystems, typischerweise über ein Level 1 - Automatisierungssystem, vor. Dies erfolgt beispielsweise nach Modellvorrechnungen auf Basis von aktuell verwendeten / verfügbaren Einsatzstoffen und von Zielvorgaben einer zu produzierenden Stahlsorte - deren Ziele und Produktionsschemata, bspw. Blasschemata, Bodenspülschemata, Zugabeschemata, etc..

Durch die chemischen Reaktionen entsteht beim Stahlherstellungsprozess bspw. in einem Sauerstoff-Konverter unter anderem CO- bzw. CO₂ als Prozessabgas, das gezielt mittels Primarentstaubungsanlage abgesaugt wird. Zusätzlich sind auch Partikel der Einsatzstoffe im Abgas enthalten. Das Abgas wird in der Entstaubungsanlage gereinigt und im besten Fall als kalorisches Gas wiederverwendet. Die Entstaubungsanlage umfasst beispielsweise ein Rohrsystem mit einer Filteranlage und einer Abzugsvorrichtung, beispielsweise einem Saugzuggebläse.

Während des Chargierens von Zuschlagsstoffen in den Konverter kann es vorkommen, dass Teile der Zuschlagsstoffe unmittelbar bei der Zugabe abgesaugt werden. Diese Menge an Zuschlagsstoffen wird folglich im Prozess nicht aktiv. Die tatsächlich abgesaugte Menge an Zuschlagsstoffen ist unbekannt. Sie ist abhängig vom aktuellen Volumenstrom der Entstaubungsanlage und vom Feinanteil der chargierten Zuschlagsstoffe. Der Level 2 Prozessrechner rechnet auf Basis der aktuell verwendeten Zuschlagsstoffe. Es wird somit die tatsächlich verwogene Menge an Zuschlagsstoffen berücksichtigt. Da die Menge der abgesaugten Zuschlagsstoffe derzeit unbekannt ist, kann diese bei den Berechnungen nicht berücksichtigt werden. Durch den Einsatz inkorrekter Mengen der Zuschlagsstoffe können die tatsächlichen Prozessergebnisse von den gewünschten Zielwerten abweichen. Derzeit können nur experimentell ermittelte oder auf Basis von Erfahrungswerten ermittelte Korrekturwerte für die Zuschlagstoffe verwendet werden. Die tatsächlich ausgetragenen Zuschlagstoffe können aktuell nicht bestimmt werden.

Folglich kommt es zu Abweichungen der Modellvorrechnungen zu den tatsächlichen gemessenen Werten und damit zur Abweichung von den gewünschten Zielgrößen. Die wichtigsten Zielgrößen beim LD-Verfahren sind Temperatur, Kohlenstoff- und Phosphorgehalt.

### Zusammenfassung der Erfindung

Die Aufgabe der Erfindung ist es den - über die Entstaubungsanlage - mitgerissenen variablen Feinanteil messbar zu machen und dadurch den Prozess effizienter zu gestalten.

Die Aufgabe wird gelöst durch ein Analysesystem welches eine Zuführeinheit, zum Zuführen vom abgetrennten Staub zu einem Materialanalysegerät aufweist. Das Materialanalysegerät ist zum Bestimmen der Anteile der im abgetrennten Staub enthaltenen Stoffe, insbesondere der Zuschlagsstoffe, ausgelegt. Das Analysesystem umfasst weiters eine Masseermittlungsvorrichtung, welche zur Bestimmung einer Masse des abgetrennten Staubes ausgestaltet ist. Eine Auswerteeinrichtung, welche mit dem Materialanalysegerät, der Masseermittlungsvorrichtung und der Zuführeinheit verbunden ist, ermittelt die Zuschlagstoffmasse, die im abgetrennten Staub enthalten ist.

Die Grundidee der Erfindung ist es, jenen Anteil der Zuschlagsstoffe zu quantifizieren, der nicht in die Schmelze fällt, sondern der über die Entstaubungsanlage abgesaugt wird. In der Entstaubungsanlage wird der Staub aus dem Rohgas gefiltert. Der Staub selbst wird hierzu je nach Bauweise der Entstaubungsanlage über Gravitationsabscheidung, elektrostatische Abscheidung, Gaswäsche oder auch Mischsysteme abgeschieden. Durch das Ausführen des Staubes aus einer Entstaubungsanlage lässt sich die Massenverteilung der aus dem Prozess abgeschiedenen Zuschlagstoffe - mittels des beschriebenen Analysesystems bestimmen. In der Erfindung werden einerseits die im Staub enthaltenen Stoffe, insbesondere die eingesetzten Zuschlagstoffe, analysiert, andererseits wird die gesamte Staubmenge eines Hauptprozesses ermittelt. Durch diese Messungen kann die Masse eines Zuschlagsstoffes als Anteil des Gesamtstaubes ermittelt oder abgeschätzt werden. Die Kenntnis der zugegebenen Zuschlagsstoffe über die Materialzugaben des Hauptprozesses und die detektierte Masse des jeweiligen Zuschlagsstoffes in den Stäuben lässt eine sehr aktuelle Verlustrate für jeden Zuschlagsstoff über die Entstaubungsanlage ermitteln oder abschätzen. Diese Verlustrate kann in weiterer Folge als direkter Parameter für den zeitlich darauffolgenden Hauptprozess verwendet werden und so die Qualität einer Zuschlagsstoffregelung und damit einer Modellberechnung erhöhen.

Falls es mehrere Zugaben eines Materials innerhalb eines Hauptprozesses gibt, kann, im bevorzugten Fall, bereits bei der nächsten Zugabe nach Bestimmung der flüchtigen Feinanteile einer oder mehrerer vorangegangener Zugaben reagiert werden.

Da dem Prozess wie beschrieben verschiedene Zuschlagstoffe zu unterschiedlichen Zeiten zugeführt werden können, kann auch eine Massenverteilung zeitabhängig ermittelt werden. Durch das Analysesystem wird eine detaillierte Analyse welche Zuschlagstoffe, in welcher Menge und/oder wann - ohne Reaktion im Prozess ausgeworfen werden, ermöglicht. Dieses Wissen kann bevorzugt dafür verwendet werden, um aus gesammelten historischen Daten Mehrmengen der Zuschlagsstoffe zu bestimmen, die zugegeben werden müssen, um den Prozess unter Verwendung der gewünschten Reaktionsmengen durchzuführen. Erlaubt der Prozess eine Mengenkorrektur zur Prozesslaufzeit, so kann durch Zugabe der bestimmten Fehlmengen der Aufwand einer Ergebniskorrektur im Rahmen sekundärmetallurgischer Prozesse reduziert bzw. vermieden werden.

Bei einer vorteilhaften Ausführung der erfindungsgemäßen Vorrichtung wird die Entstaubungsanlage so betrieben, dass möglichst große Anteile der Feinanteile mit bekannter Zuordnung zum zeitlichen Materialzugabeverlauf vom Abgasstrom abgetrennt und unmittelbar dem Analysegerät zugeführt werden. Insbesondere soll die Ausführung derart gestaltet sein, dass Staubabscheidungen zeitlich aufeinanderfolgender Schmelzen eindeutig zu den jeweiligen Schmelzen zugeordnet werden können. Bei Entstaubungssystem, bei denen der Austrag direkt auf die Abscheidung folgt, ist keine besondere Fahrweise notwendig (First-in-first-out-Prinzip). Bei Schlauchfilteranlagen oder elektrostatischen Abscheidern wird Staub am Abscheidungsorgan angelagert und wird erst durch gezielte Abreinigung dem Austrag zugeführt. Bei solchen Systemen soll eine Austragung vor und nach der Materialzugabe bzw. Schmelze stattfinden, um eine Zuordnung zu ermöglichen. Aus prozesstechnischen Gründen könnte es möglich sein, dass nicht der gesamte Staub in der Entstaubungsanlage ausgetragen werden kann. In diesem erfolgt zielgerichtet der Austrag nur aus Teilen der Entstaubungsanlage.

In einer vorteilhaften Ausführungsform ist die Zuführeinheit ein Kettenförderer oder ein Bandförderer, insbesondere ein Gurtförderer.

Ein Bandförderer oder Kettenförderer eignet sich besonders gut, um den Staub zum Materialanalysegerät zu fördern.

Eine bevorzugte Ausführungsform sieht vor, dass das Materialanalysegerät auf Basis eines laser-spektroskopisches Verfahrens, eines Röntgenfluoreszenzverfahrens und/oder einer Massenspektrometrie arbeitet.

Die bekannten laser-spektroskopischen Verfahren LIBS (laser induced breakdown spectroscopy) und LIPS (laser induced plasma spectroscopy) sind bewährte Methoden zur Materialerkennung. Dabei wird mit einem fokussierten, üblicherweise gepulsten Laserstrahl auf dem Zielmaterial eine Reaktion herbeigeführt. Dabei entsteht ein Plasma, bei dessen Abklingen optische Strahlung mit einem für ein analysiertes Material und vorherrschende Umgebungsbedingungen charakteristischen Spektrum emittiert wird. Durch Aufnahme dieser Strahlung und Auswertung dieser Messergebnisse lässt sich ein Zielobjekt stofflich charakterisieren. Da dieser Prozess sehr rasch abläuft - typischerweise in wenigen Millisekunden - ist der Einsatz dieses Verfahrens auf schnell laufenden Förderbändern möglich.

Beim Röntgenfluoreszenzverfahren (XRF, X-Ray Fluorescence) wird das zu untersuchende Material durch hochenergetische Röntgenstrahlung zu einer Reaktion angeregt, deren Ergebnis die Emission von niedrigenergetischerer Strahlung ist, deren Wellenlangenspektrum für eine untersuchte Materialzusammensetzung charakteristisch ist. Diese Methode wird bereits zur Bestimmung der Materialzusammensetzung von Fördergütern verwendet. Dieses Verfahren lässt sich ebenfalls zum Beispiel auf Förderbändern einsetzen.

Die Massenspektrometrie lässt sich - im Gegensatz zu den beiden zuvor genannten Verfahren - nicht als fortlaufende Messung implementieren, sondern erfordert ein Nehmen von Materialproben aus dem Fördergutstrom. Eine entnommene Materialprobe wird schließlich zur Analyse aufbereitet und mit einem Massenspektrometer auf ihre Materialzusammensetzung hin untersucht.

Ein vorteilhafte Ausführungsform sieht vor, dass die Masseermittlungsvorrichtung mittels eines Bunker, welcher über eine Wiegevorrichtung verfügt, ausgeführt ist oder die Masseermittlungsvorrichtung mithilfe einer optischen Sensoreinrichtung das Volumen des Staubes bestimmt und anhand der Zusammensetzung des Staubes die Auswerteeinrichtung eine Dichte des Staubes ermitteln kann und aus dem Volumen und der Dichte die Masse des Staubes berechnet.

Eine zweckmäßige Ausführung sieht vor, dass die Masseermittlungsvorrichtung in Form einer Bandwaage implementiert wird. Eine Bandwaage erlaubt eine Masseermittlung in Echtzeit.

Eine besonders vorteilhafte Ausführungsform sieht vor, dass die Auswerteeinrichtung derart eingerichtet ist, um die ermittelte Zuschlagstoffmasse an eine Prozesssteuerung und/oder Regelung des metallurgischen Prozesses zu übermitteln.

Dadurch ist gewährleistet, dass die Prozesssteuerung unmittelbar auf die ausgetragenen Zuschlagstoffe reagieren kann und Gegenmaßnahmen veranlassen kann.

In einer ganz besonders vorteilhaften Ausführungsform verfügt der Bandförderer über eine Verteileinheit, welche den Staub über die Breite dünnschichtig verteilt.

Dadurch wird eine dünnschichtige Beschickung zum Materialanalysegerät zu gewährleistet, wodurch die Anforderungen an das Analysegerät reduziert werden, und die Qualität des Messergebnisse steigt.

Die Aufgabe wird auch durch ein Verfahren gelöst. Im Verfahren wird der Staub mithilfe einer Zuführeinheit einem Materialanalysegerät zugeführt. Durch das Materialanalysegerät wird die Zusammensetzung und die Anteile des Staubes analysiert und das Analyseergebnis an eine Auswerteeinrichtung übergeben. Eine Masse des Staubs wird mithilfe einer Masseermittlungsvorrichtung bestimmt und die Masse wird an die Auswerteeinrichtung übermittelt. Die Auswerteeinrichtung ermittelt für den - zumindest einen - Zuschlagstoff die Zuschlagstoffmasse. Durch das Verfahren ergeben sich dieselben Vorteile wie bereits beim Analysegerät erläutert.

In einer vorteilhaften Ausführungsform ist die Zuführeinheit ein Kettenförderer, ein Bandförderer, insbesondere ein Gurtförderer.

Ein Bandförderer eignet sich besonders gut, um den Staub zum Materialanalysegerät zu fördern.

Eine bevorzugte Ausführungsform sieht vor, dass das Materialanalysegerät auf Basis eines laser-spektroskopisches Verfahrens, eines Röntgenfluoreszenzverfahrens und/oder einer Massenspektrometrie arbeitet.

Eine vorteilhafte Ausführungsform sieht vor, dass die Masseermittlungsvorrichtung mittels eines Bunkers, welcher über eine Wiegevorrichtung verfügt, ausgeführt ist oder die Masseermittlungsvorrichtung mithilfe einer optischen Sensoreinrichtung das Volumen des Staubes bestimmt und anhand der Zusammensetzung des Staubes die Auswerteeinrichtung eine Dichte des Staubes ermitteln kann und aus dem Volumen und der Dichte die Masse des Staubes berechnet.

Eine zweckmäßige Ausführung sieht vor, dass die Masseermittlungsvorrichtung über eine Bandwaage erfolgt.

Eine besonders vorteilhafte Ausführungsform sieht vor, dass die Auswerteeinrichtung derart eingerichtet ist, um die ermittelte Zuschlagstoffmasse an eine Prozesssteuerung und/oder Regelung des metallurgischen Prozesses zu übermitteln.

Dadurch ist gewährleistet, dass die Prozesssteuerung unmittelbar auf die ausgetragenen Zuschlagstoffe reagieren kann und Gegenmaßnahmen veranlassen kann.

In einer ganz besonders vorteilhaften Ausführungsform verfügt der Bandförderer über eine Verteileinheit, welche den Staub über die Breite dünnschichtig verteilt.

Dadurch wird eine dünnschichtige Beschickung zum Materialanalysegerät zu gewährleistet.

Des Weiteren wird die Aufgabe durch eine metallurgische Anlage gelöst, welche ein zuvor beschriebenes Analysesystem umfasst.

Eine vorteilhafte Ausführungsform sieht vor, dass die metallurgische Anlage einen Konverter zum Durchführen eines Sauerstoff-Konverter-Prozesses - auch als LD (Linz Donawitz) Verfahren bekannt - oder AOD (Argon Oxygen Decarburization) Verfahrens, einen Elektrolichtbogenofen, einen Smelter oder einen Hochofen umfasst.

Ein Smelter ist ein elektrisch betriebener Ofen zur Herstellung von flüssigem Metall, insbesondere Roheisen. Als Eingangsstoff in den Smelter wird beispielsweise direkt reduziertes Eisen verwendet. Bei der Erzeugung von Roheisen durch den Smelter entsteht zusätzlich Schlacke.

### Kurze Beschreibung der Zeichnungen

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung eines Ausführungsbeispiels, das im Zusammenhang mit den Zeichnungen näher erläutert wird. Dabei zeigen:

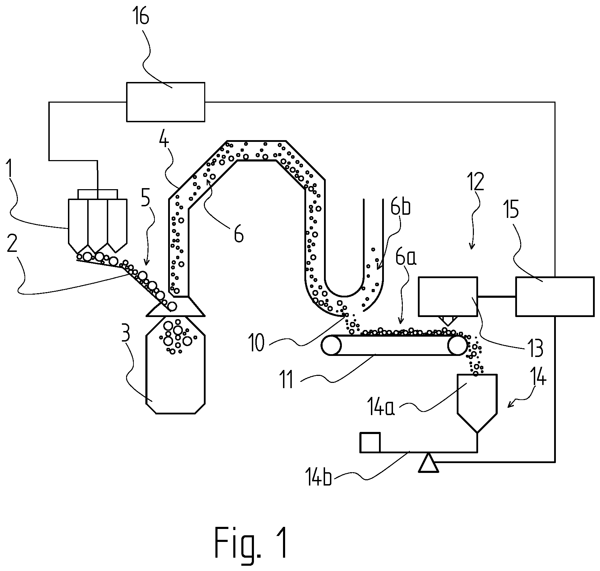

Fig 1 eine schematische Darstellung einer metallurgischen Anlage mit einem Analysesystem für ausgetragener Zuschlagstoffe,

### Beschreibung der Ausführungsformen

In der Fig. 1 ist eine schematische Darstellung einer metallurgischen Anlage mit einem Analysesystem 12 für ausgetragene Zuschlagstoffe 5 gezeigt. Die Zuschlagstoffe 5 werden von einer Prozesssteuerung 16 vorgegeben. Aus einem Bunker 1 werden die von der Prozessteuerung 16 vorgegebenen Zuschlagstoffe 5 einem Konverter 3 zur Stahlherstellung - mithilfe eines Beschickungssystems 2 - zugegeben. Beim Zugeben der Zuschlagstoffe 5 wird ein Teil über einen mit Staub 6a beladenen Abgasstrom 6 in einem Absaugsystem 4 ausgetragen. Der Staub 6a beinhaltet einen Anteil des Zuschlagstoffes 5, welche in einer Abscheidungssystem 10 vom Abgasstrom 6 abgetrennt wird und der Staub 6a wird einem Analysesystem 12 zugeführt. In einem ersten Schritt wird im Analysesystem 12 der Staub 6a über eine Zuführeinheit 11 einem Materialanalysegerät 13 zugeführt. Das Materialanalysegerät 13 bestimmt die Materialzusammensetzung des Staubes 6a. Das Materialanalysegerät 13 arbeitet beispielsweise anhand eines laser-spektroskopisches Verfahrens, ein Röntgenfluoreszenzverfahrens und/oder einer Massenspektrometrie. Das Materialanalysegerät 13 kann den relativen Anteile zwischen dem Anteil des Zuschlagstoffes 5, welcher im ausgetragenen Staub 6a enthalten ist, bestimmen. Zusätzlich zur Bestimmung der Materialzusammensetzung des Staubes 6a wird dieser einer Massenermittlungsvorrichtung 14 hinsichtlich Menge bestimmt, beispielsweise mit einer Bunkerwaage nach bzw. Bandwaage vor dem Materialanalysegerät 13. In der gezeigten Ausführungsform handelt es sich hierbei um einen Bunker 14a mit einer Wiegevorrichtung 14b. Die Wiegevorrichtung 14b kann die gesamte Masse und den Massenstrom des abgetrentem Staubes 6a bestimmen. Im Staub 6a sind Zuschlagstoffe 5 enthalten, deren Teilmassenströme mit Hilfe der Messdaten aus der Wiegevorrichtung 14b in der Auswerteeinrichtung 15 ermittelt werden. Die Zuschlagsstoffmassenströme werden von der Auswerteeinrichtung 15 der Prozesssteuerung 16 übermittelt. Dies ermöglicht die relativen Anteile der Zuschlagsstoffe 5, die über den Abgasstrom dem Prozess verloren gehen kontinuierlich zu bestimmen und so kann der Zuschlagsstoff 5 charakterisiert werden, welcher im Prozess zur Stahlherstellung verblieben ist. Dadurch können in einem nachfolgenden Prozessdurchlauf die zugeführten Zuschlagstoffmengen entsprechen korrigiert werden. Es ist aber auch denkbar, dass Gewichte der verloren gegangenen Zuschlagsstoffe des aktuellen Prozessdurchlaufes ermittelt werden und bei Bedarf im aktuellen Prozessdurchlauf berücksichtigt bzw. korrigiert werden.

Obwohl die Erfindung im Detail durch die bevorzugten Ausführungsbeispiele näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

### Bezugszeichenliste

- 1: Bunker
- 2: Beschickungssystem
- 3: Konverter
- 4: Absaugsystem
- 5: Zuschlagstoffe
- 6: Abgasstrom
- 6a: Staub
- 10: Abscheidungssystem
- 11: Zuführeinheit
- 12: Analysesystem
- 13: Materialanalysegerät
- 14: Massenermittlungsvorrichtung
- 14a: Bunker
- 14b: Wiegevorrichtung
- 15: Auswerteeinrichtung
- 16: Prozesssteuerung

## Patentansprüche

1. Analysesystem (12) zur Ermittlung zumindest einer Zuschlagstoffmasse von einem ausgetragenen Zuschlagsstoff (5) eines metallurgischen Prozesses, wobei dem Analysesystem (12) ein durch ein Abscheidungssystem (10) von einem Abgas abgetrennter Staub zugeführt werden kann, **dadurch gekennzeichnet, dass** das Analysesystem (12),
- eine Zuführeinheit (11), zum Zuführen vom abgetrennten Staub (6a) zu einem Materialanalysegerät (13),
- das Materialanalysegerät (13), zum Bestimmen der Anteile der im abgetrennten Staub (6a) enthaltenen Stoffe, insbesondere der Zuschlagsstoffe (5),
- eine Masseermittlungsvorrichtung (14), zur Bestimmung einer Masse des abgetrennten Staubes (6a),
- eine Auswerteeinrichtung (15), welche mit dem Materialanalysegerät (13), der Masseermittlungsvorrichtung (14) und der Zuführeinheit (11) verbunden ist, um die Zuschlagstoffmasse, die im abgetrennten Staub (6a) enthalten ist, zu ermitteln,
umfasst.

2. Analysesystem (12) zur Ermittlung von ausgetragenen Zuschlagsstoffen (5) eines metallurgischen Prozesses nach Anspruch 1, **dadurch gekennzeichnet, dass** die Zuführeinheit (11) ein Kettenförderer oder ein Bandförderer, insbesondere ein Gurtförderer, ist.

3. Analysesystem (12) zur Ermittlung von ausgetragenen Zuschlagsstoffen (5) eines metallurgischen Prozesses nach Anspruch 1 oder 2, **dadurch gekennzeichnet, dass** das Materialanalysegerät (13) ein laser-spektroskopisches Verfahren, ein Röntgenfluoreszenzverfahren und/oder eine Massenspektrometrie ausführt.

4. Analysesystem (12) zur Ermittlung von ausgetragenen Zuschlagsstoffen (5) eines metallurgischen Prozesses nach einem der Ansprüche 1 - 3, **dadurch gekennzeichnet, dass** die Masseermittlungsvorrichtung (14) mittels eines Bunker, welcher über eine Wiegevorrichtung verfügt, ausgeführt ist oder die Masseermittlungsvorrichtung (14) mithilfe einer optischen Sensoreinrichtung das Volumen des Staubes (6a) bestimmt und anhand der Zusammensetzung des Staubes (6a) die Auswerteeinrichtung (15) eine Dichte des Staubes (6a) ermitteln kann und aus dem Volumen und der Dichte die Masse des Staubes (6a) berechnet.

5. Analysesystem (12) zur Ermittlung von ausgetragenen Zuschlagsstoffen (5) eines metallurgischen Prozesses nach einem der Ansprüche 1 - 4, **dadurch gekennzeichnet, dass** die Masseermittlungsvorrichtung (14) über eine Bandwaage erfolgt.

6. Analysesystem (12) zur Ermittlung von ausgetragenen Zuschlagsstoffen (5) eines metallurgischen Prozesses nach einem der Ansprüche 1 - 5, **dadurch gekennzeichnet, dass** die Auswerteeinrichtung (15) derart eingerichtet ist, um die ermittelte Zuschlagstoffmasse an eine Prozesssteuerung (16) und/oder Regelung des metallurgischen Prozesses zu übermitteln.

7. Analysesystem (12) zur Ermittlung von ausgetragenen Zuschlagsstoffen (5) eines metallurgischen Prozesses nach einem der Ansprüche 2 - 6, **dadurch gekennzeichnet, dass** der Bandförderer oder Kettenförderer über eine Verteileinheit verfügt, welche den Staub über eine Breite dünnschichtig verteilt.

8. Verfahren zur Ermittlung zumindest einer Zuschlagstoffmasse von zumindest einem ausgetragenen Zuschlagsstoff (5) eines metallurgischen Prozesses, wobei dem metallurgischen Prozess mittels eines Absaugsystem (4) ein mit Staub (6a) beladener Abgasstrom abgezogen wird und an einem Abscheidungssystem (10) der Staub (6a) vom Abgasstrom (6) abgetrennt wird, **dadurch gekennzeichnet, dass** der Staub (6a) mithilfe einer Zuführeinheit (11) einem Materialanalysegerät (13) zugeführt wird, durch das Materialanalysegerät (13) wird die Zusammensetzung und die Anteile des Staubes analysiert und das Analyseergebnis an eine Auswerteeinrichtung (15) übergeben, wobei eine Masse des Staub mithilfe einer Masseermittlungsvorrichtung (14) bestimmt wird und die Masse an die Auswerteeinrichtung (15) übermittelt wird, wobei die Auswerteeinrichtung (15) für den zumindest einen Zuschlagstoff (5) die Zuschlagstoffmasse ermittelt.

9. Verfahren zur Ermittlung zumindest einer Zuschlagstoffmasse von zumindest einem ausgetragenen Zuschlagsstoff (5) eines metallurgischen Prozesses nach Anspruch 8, **dadurch gekennzeichnet, dass** die Zuführeinheit (11) ein Kettenförderer oder ein Bandförderer, insbesondere ein Gurtförderer, ist.

10. Verfahren zur Ermittlung zumindest einer Zuschlagstoffmasse von zumindest einem ausgetragenen Zuschlagsstoff (5) eines metallurgischen Prozesses nach Anspruch 8 - 9, **dadurch gekennzeichnet, dass** das Materialanalysegerät (13) ein laser-spektroskopisches Verfahren, ein Röntgenfluoreszenzverfahren Verfahren und/oder eine Massenspektrometrie ausführt.

11. Verfahren zur Ermittlung zumindest einer Zuschlagstoffmasse von zumindest einem ausgetragenen Zuschlagsstoff (5) eines metallurgischen Prozesses nach Anspruch 8 - 10, **dadurch gekennzeichnet, dass** die Masseermittlungsvorrichtung (14) mittels eines Bunker (14a), welcher über eine Wiegevorrichtung (14b) verfügt, ausgeführt ist oder die Masseermittlungsvorrichtung (14) mithilfe einer optischen Sensoreinrichtung das Volumen des Staubes (6a) bestimmt und anhand der Zusammensetzung des Staubes die Auswerteeinrichtung (15) eine Dichte des Staubes (6a) bestimmt und aus dem Volumen und der Dichte die Masse des Staubes (6a) berechnet.

12. Verfahren zur Ermittlung zumindest einer Zuschlagstoffmasse von zumindest einem ausgetragenen Zuschlagsstoff (5) eines metallurgischen Prozesses nach Anspruch 8 -11, **dadurch gekennzeichnet, dass** die Massenermittlungsvorrichtung (14) eine Bandwaage ist.

13. Verfahren zur Ermittlung zumindest einer Zuschlagstoffmasse von zumindest einem ausgetragenen Zuschlagsstoff (5) eines metallurgischen Prozesses nach Anspruch 8 - 12, **dadurch gekennzeichnet, dass** die Auswerteeinrichtung (15) die ermittelte Zuschlagstoffmasse an eine Prozesssteuerung (16) und/oder Regelung des metallurgischen Prozesses übermittelt.

14. Metallurgische Anlage zum Herstellen eines flüssigen Metalls umfassend ein Beschickungssystem (2) für zumindest einen Zuschlagstoff (5), eine Entstaubungsanlage mit einem Abscheidungssystem (10) für ausgetragenem Staub (6a) und einer Prozesssteuerung (16) und/oder Prozessregelung, **dadurch gekennzeichnet, dass** diese ein Analysesystem (12) nach einem der Ansprüche 1-7 umfasst.

15. Metallurgische Anlage zum Herstellen eines flüssigen Metalls nach Anspruch 14, **dadurch gekennzeichnet, dass** die metallurgische Anlage einen Konverter zum Durchführen eines LD-Verfahrens oder AOD Verfahrens, einen Elektrolichtbogenofen, einen Smelter oder einen Hochofen umfasst.
